# EUROPÄISCHE PATENTSCHRIFT

(11) **EP 0 670 721 B1**
(45) Veröffentlichungstag und Bekanntmachung des Hinweises auf die Patenterteilung: **11.08.1999**
(21) Anmeldenummer: 93920843.5
(22) Anmeldetag: 30.09.1993
(51) Int. Cl.: A61K 31/37, A61K 31/35, A61K 31/185, A61K 31/70

(54) **ARZNEIMITTEL ZUR BEHANDLUNG UND PROPHYLAXE VON KNOCHEN- UND GELENKSERKRANKUNGEN**
DRUG FOR TREATMENT AND PROPHYLAXIS OF BONE AND JOINT DISEASES
MEDICAMENT UTILISE DANS LE TRAITEMENT ET LA PROPHYLAXIE DES MALADIES OSSEUSES ET ARTICULAIRES

(43) Veröffentlichungstag der Anmeldung: 13.09.1995
(73) Patentinhaber: Fricker, Christian, CH-8165 Schöfflisdorf (CH)
(72) Erfinder: Fricker, Christian, CH-8165 Schöfflisdorf (CH)
(74) Vertreter: VOSSIUS & PARTNER
(86) Internationale Anmeldenummer: EP9302668
(87) Internationale Veröffentlichungsnummer: WO9508992

(56) Entgegenhaltungen:
- EP-A- 0 198 965
- CH-A- 682 716
- BR. J. EXP. PATHOL. Bd. 57, Nr. 4 , 1976 Seiten 713 - 721 N.B. PILLER 'The action of the benzopyrones on an experimental model of lymphoedema: a contribution to their mode of action.'
- LYMPHOLOGY Bd. 18, Nr. 1 , 1985 Seiten 37 - 45 J.R. CASLEY-SMITH ET AL. 'The effects of calcium dobesilate on acute lymphedema (with and without macrophages), and on burn edema.'
- ASSOCIATION OF OPERATION ROOM NURSES JOURNAL Bd. 56, Nr. 5 , 1992 Seiten 904 - 911 L.P. GOOD 'Compartment syndrome- a closer look at etiology, treatment.'

## Beschreibung

Die Erfindung betrifft die Verwendung von Benzopyronen und von Dobesilat-Calcium zur Herstellung eines Arzneimittels zur Behandlung und Prophylaxe von Knochen- und Gelenkserkrankungen, deren Pathophysiologie dem Prinzip des Kompartment-Syndroms entspricht.
Die Erfindung betrifft des weiteren auch ein Verfahren zur Behandlung und Prophylaxe derartiger Erkrankungen, wobei eine wirksame Menge der vorstehend genannten Verbindungen einem Säuger verabreicht wird.

Die Verwendung von Benzopyronen zur Linderung begleitender Ödeme ist in der Literatur beschrieben: auch die AU-B-25 216/84 lehrt und beansprucht derartige Behandlungen. In der US-A-3 509 207 wird der Einsatz von Dobesilat-Calcium zum Stillen von Blutungen beschrieben.

Kompartment-Syndrome zeichnen sich aus durch einen schmerzhaften Druckanstieg in anatomisch vorgegebenen, begrenzt dehnbaren Räumen. Im Gegensatz dazu sind Ödeme durch eine schmerzlose Schwellung charakterisiert.
Kompartment-Syndrome beschränken sich auf Gewebe, deren Volumen infolge zirkulatorischer Störungen nicht vergrößert werden kann, d.h. in denen sich auf Grund anatomischer Gegebenheiten kein Ödem ausbilden kann. Das Konzept des Kompartment-Syndroms kann entsprechend dem Geschehen in den Muskellagen auch auf Knochen, Bänder und auf die Gelenkkapsel übertragen werden.
Kennzeichnend für das Kompartment-Syndrom ist der unphysiologisch erhöhte Druck in den erkrankten Geweben:
Eine erhöhte Gefäßpermeabilität führt zu einem vermehrten Flüssigkeitseinstrom in das Interstitium sowie zur Freisetzung von Substanzen zum Aufbau eines osmotischen Gradienten in der gleichen Richtung. Beide Faktoren führen im Interstitium zu einer positiven Flüssigkeitsbilanz. Knochen, Bänder und Gelenkkapsel bilden für die in ihnen eingebetteten Strukturen eine begrenzende Hülle. Ihr Volumen kann sich nicht ausdehnen, so daß ein interstitieller Druckanstieg resultiert.
Die Folge ist eine Beeinträchtigung der Mikrozirkulation. Es entstehen hämodynamisch stillgelegte Areale, in denen die Zirkulationsstörung im Sinne eines circulus vitiosus aufrecht erhalten wird. Durch die verzögerte Mikrozirkulation wird das Sauerstoffangebot reduziert. Das Sauerstoffdefizit führt zur anaerobern Glykolyse und zur Azidose. Die Folgen für die Funktion und die Struktur der Zellen sind vielfältig, wobei die Schädigungsskala schließlich beim Zelltod endet. Der Sauerstoffmangel beeinflußt die Regulation des interstitiellen Drucks im Sinne einer negativen Rückkopplung:
- Die Gefäßpermeabilität wird erhöht, d.h. es findet ein vermehrter Flüssigkeitseinstrom ins Interstitium statt.
- Es werden zusätzlich Substanzen für den Aufbau eines osmotischen Gradienten in Richtung Interstitium frei (Abb. 1).

Es ist bekannt, daß bei degenerativen Gelenkserkrankungen (z.B. Koxarthrose, Gonarthrose, Patellar Pain, Metatarsus Köpfchennekrose) in den am Gelenk beteiligten Knochen ein erhöhter Druck vorliegt. Es ist ferner bekannt, daß bei der Podotrochlose des Pferdes (Strahlbeinlahmheit) der Druck im Strahlbein erhöht ist. Auf Grund der Analogie darf angenommen werden, daß der Druck in der Gelenkkapsel ebenfalls erhöht ist.

Wesentliche Grundlage für den neuen Anwendungsbereich von Benzopyronen und Dobesilat-Calcium bildet die Übertragung des Kompartment-Syndrom-Konzeptes auf mit Druckerhöhung einhergehende Erkrankungen von Knochen und Gelenken bei Mensch und Tier.

Erfindungsgemäß konnte festgestellt werden, daß bei degenerativen Gelenkserkrankungen in den Epiphysen bei am Gelenk beteiligten Knochen ein pathologisch erhöhter Innendruck vorliegt. Analog zur Pathophysiologie des Kompartmentsyndroms wird der intraossäre Druckanstieg durch eine Anreicherung von osmotisch aktiven Substanzen in Intestitium verursacht. In Anlehnung an die vorgestellte Pathophysiologie kann somit erstmals eine kausale Therapie für degenerative Gelenkserkrankungen bereitgestellt werden.
Benzopyrone und Dobesilat-calcium vermögen durch Stabilisierung der Kapillarwände einerseits und durch Proteinresorption andererseits den Druck im erkrankten Kompartment abzubauen. Der Druckabbau ermöglicht in der Folge eine Normalisierung der Mikrozirkulation und somit eine Gesundung der betroffenen Gewebe.
Wie eigene, kontrollierte Versuche zeigen, eignen sich Benzopyrone wie auch Dobesilat-Calcium zur kausalen Therapie dieser auf innerem Überdruck in Knochen und Gelenken beruhenden Erkrankungen. Dies trifft insbesondere in der Human- und Veterinärmedizin für den Problemkreis der Osteoarthrose und in der Veterinärmedizin für die Podotrochlose des Pferdes zu.

Für die erfindungsgemäße Verwendung sind folgende Benzopyrone besonders geeignet.
1,2-Benzopyron-Derivate der allgemeinen Formel I 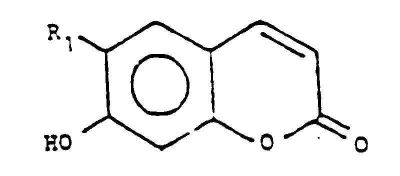 in der R₁ Wasserstoff, Halogen oder eine Hydroxy-, Sulfonyl-, Alkyl-, Hydroxyalkyl-, Acyloxy-, Alkoxy- oder Benzylgruppe oder ein Glycosidrest ist, sowie 1,4-Benzopyrone der allgemeinen Formel II 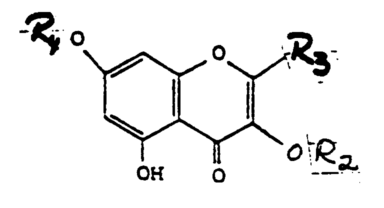 in der R² und R⁴ unabhängig voneinander Wasserstoff, Halogen oder eine Hydroxy-, Sulfonyl-, Alkyl-, Hydroxyalkyl-, Acyloxy-, Alkoxy- oder Benzylgruppe oder ein Glykosidrest sind, und
R³ Wasserstoff oder einen Phenylrest der allgemeinen Formel 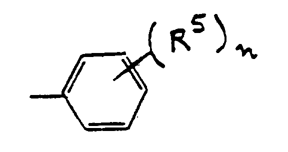 bedeutet, in der R⁵ ein Halogenatom, eine Hydroxy-, Sulfonyl-, Alkyl-, Hydroxyalkyl-, Acyloxy- oder Alkoxygruppe ist und n Werte von 0 bis 3 annehmen kann.

Besonders bevorzugt sind folgende Benzopyronverbindungen: 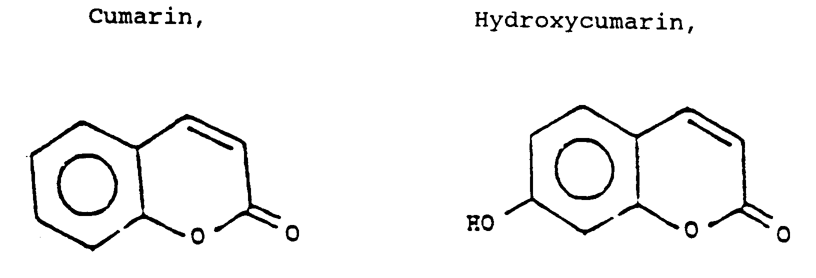 Rutin, Monoxerutin, Troxerutin 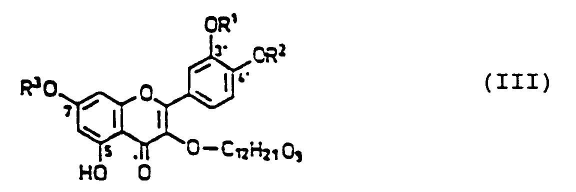
R¹ = R² = R³ = H; Rutein
R¹ = R² = H, R³ = (CH₂)₂-OH Monocerutin
R¹ = R² = R³ = (CH₂)₂-OH Troxerutin
sowie die Verbindung Diosnin

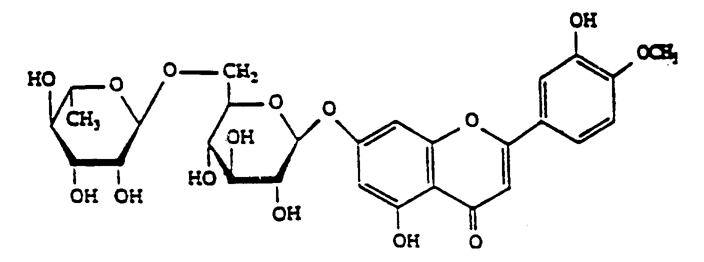

In den vorstehend genannten allgemeinen Formeln I und II bedeuten "Alkyl", "Hydroxyalkyl", "Acyloxy" und "Alkoxy" Reste mit 1 bis 6, vorzugsweise 1 bis 3 Kohlenstoffatome. Unter Halogen versteht man Fluor, Chlor oder Brom.

Als Glycoside kommen beispielsweise α- und β-D-Glucoside und das β-D-Glucopyranasid in betracht.

Die erfindungsgemäßen Verbindungen können in an sich bekannter Weise hergestellt werden, vgl. Beilstein III/IV, Bd. 18, S. 294ff. Desweiteren sind viele dieser Verbindungen, einschließlich der bevorzugten Verbindungen als Naturstoffe im Handel erhältlich. Ferner ist die synthetische Herstellung der Glucoside beispielsweise nach H. Wagner et al, Chem. Ber. Bd. 102 (1969) S. 3006 möglich. Schließlich können die erfindungsgemäßen Substanzen nach dem Fachmann bekannten Verfahren enzymatisch im Fermenter hergestellt werden.

Außer der Verwendung der Benzopyrone umfaßt die Erfindung auch die Verwendung von Dobesilat-Calcium der Formel

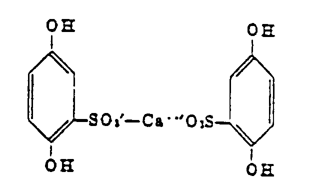

Die Herstellung dieser Verbindung wird in der US-A-3 509 207 beschrieben.

Die erfindungsgemäßen Verbindungen können in üblicher Weise oral, parenteral oder rektal verabfolgt werden. Die Dosierung hängt vom Alter, Zustand und Gewicht des Patienten sowie von der Applikationsart ab. In der Regel beträgt die tägliche Wirkstoffdosis 0,1 bis 10 mg/kg Körpergewicht bei oraler Gabe und etwa 0,1 bis 5,0 mg/Körpergewicht bei parenteraler Gabe, vorzugsweise von 0,2 bis 2,0 mg/kg Körpergewicht.

Die erfindungsgemäßen Verbindungen können in üblichen galenischen Applikationsformen fest oder flüssig angewendet werden, z,B. als Tabletten, Filmtabletten, Kapseln, Pulver, Granulate, Dragées, Suppositorien oder Lösungen. Bevorzugt sind Feststoffzubereitungen wie Depot- bzw. Retard-Dragées, Pulverzubereitungen, Granulate und Suppositorien, sowie intravenös verabreichbare Lösungen. Diese werden in üblicher Weise hergestellt. Die Wirkstoffe können dabei mit den üblichen galenischen Hilfsmitteln, wie pharmazeutisch verträglichen Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln und/oder Antioxidantien verarbeitet werden; vgl. H. Sucker et al., Pharmazeutische Technologie, Thieme-Verlag, Stuttgart, 1978. Die so erhaltenen Applikationsformen enthalten den Wirkstoff normalerweise in einer Menge von 1 bis 99 Gew.-%, vorzugsweise in einer Menge von 1 bis 10 Gew.-%.

Die nachstehenden Beispiele belegen die Wirksamkeit der erfindungsgemäßen Verwendung.

### Beispiel 1

Das 5,6-α-Benzopyron wurde in der Tagesdosis von:
1 - 4 mg/kg KGW beim Menschen,
2 - 4 mg/kg KGW beim Hund und
0,5 - 1 mg/kg KGW beim Pferd
peroral verabreicht.

### Beispiel 2

Das Dobesilat-Calcium wurde in der Tagesdosis von:
10 - 20 mg/kg KGW beim Menschen und
10 - 20 mg/kg KGW beim Hund
peroral verabreicht.

Der Therapieerfolg wurde in der Regel nach einer Behandlungsdauer von 2 - 4 Monaten festgestellt. Rezidive wurden nur selten beobachtet.

## Patentansprüche

1. Verwendung von Benzopyronen (Benzo-α-pyron und Derivate, Benzo-γ-pyron und Derivate) oder von Dobesilat-Calcium (2,5-Dihydroxybenzosulfonat) zur Herstellung eines Arzneimittels zur Behandlung und Prophylaxe von Knochen- und Gelenkserkrankungen, deren Pathophysiologie dem Prinzip des Kompartment-Syndroms entspricht.

2. Verwendung nach Anspruch 1, dadurch gekennzeichnet, daß die Benzopyrone 1,2-Benzopyron-Derivate der allgemeinen Formel 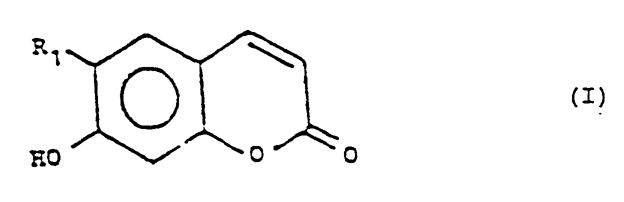 sind, in der R₁ Wasserstoff, Halogen oder eine Hydroxy-, Sulfonyl-, Alkyl-, Hydroxyalkyl-, Acyloxy-, Alkoxy- oder Benzylgruppe oder ein Glycosidrest ist.

3. Verwendung nach einem der Ansprüche 1 oder 2, dadurch gekennzeichnet, daß die Benzopyrone 1,2-Benzopyrone der Formeln 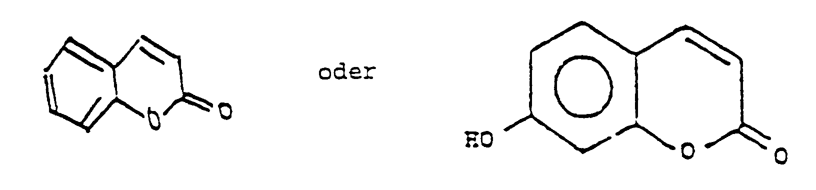 sind.

4. Verwendung nach Anspruch 1, dadurch gekennzeichnet, daß die Benzopyrone 1,4-Benzopyrone der allgemeinen Formel 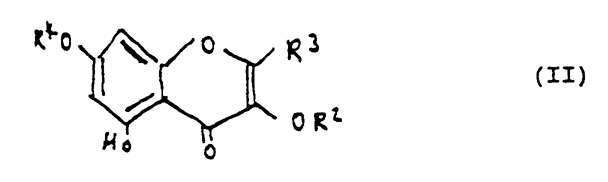 sind, in der R² und R⁴ unabhängig voneinander Wasserstoff, Halogen oder eine Hydroxy-, Sulfonyl-, Alkyl-, Hydroxyalkyl-, Acyloxy-, Alkoxy- oder Benzylgruppe oder ein Glykosidrest sind, und
R³ Wasserstoff oder einen Phenylrest der allgemeinen Formel 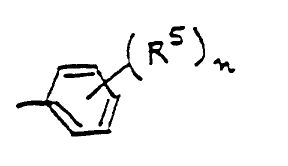 bedeutet, in der R⁵ ein Halogen, eine Hydroxy-, Sulfonyl-, Alkyl-, Hydroxyalkyl-, Acyloxy- oder Alkoxygruppe ist und n Werte von 0 bis 3 annehmen kann.

5. Verwendung nach einem der Ansprüche 1 oder 4, dadurch gekennzeichnet, daß die Benzopyron-Derivate 1,4-Benzopyron-Derivate der Formel 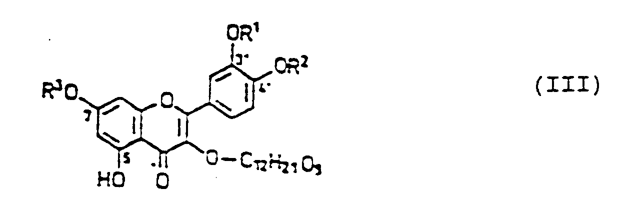 sind, in der R¹, R², R³ Wasserstoff bedeuten oder R¹ und R² Wasserstoff und R³ ein Rest (CH₂)₂-OH sind oder R¹, R² und R³ ein Rest (CH₂)₂-OH sind.

6. Verwendung nach einem der Ansprüche 1 oder 4, dadurch gekennzeichnet, daS das Benzopyron ein 1,4-Benzopyron der Formel 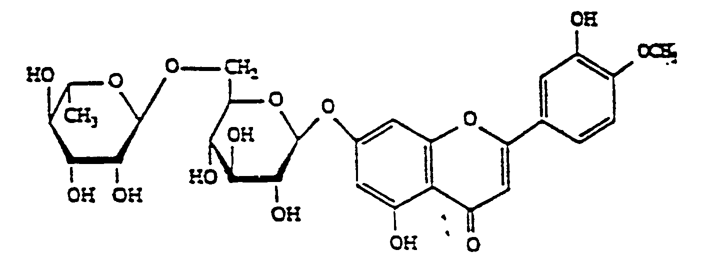 ist.

## Claims

1. Use of benzopyrones (benzo-α-pyrone and derivatives, benzol-γ-pyrone and derivatives) or of calcium dobesilate (2,5-dihydroxybenzosulfonate) for the preparation of a pharmaceutical composition for the treatment and prophylaxis of bone and joint diseases, the pathophysiology of which corresponds to the principle of the compartment syndrome.

2. The use of claim 1, wherein the benzopyrones are 1,2-benzopyrone derivatives of the general formula 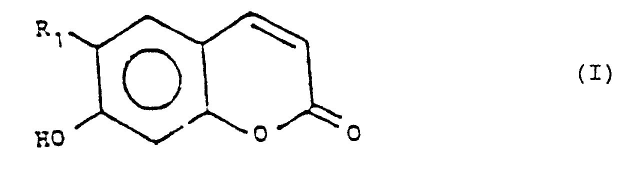 wherein R₁ is hydrogen, halogen or a hydroxy, sulfonyl, alkyl, hydroxyalkyl, acyloxy, alkoxy or benzyl group or a glycosidic residue.

3. The use of any one of claims 1 or 2, wherein the benzopyrones are 1,2-benzopyrones of the formulae

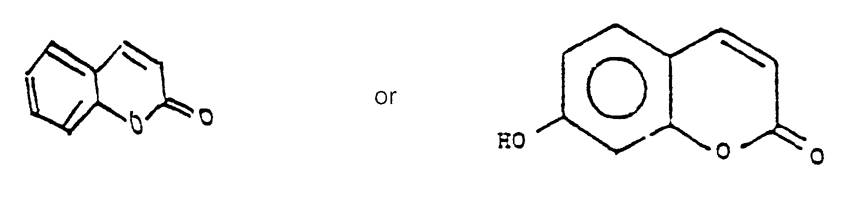

4. The use of claim 1, wherein the benzopyrones are 1,4-benzopyrones of the general formula 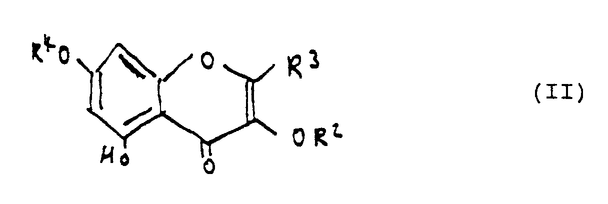 wherein R² and R⁴ are independently hydrogen, halogen or a hydroxy, sulfonyl, alkyl, hydroxyalkyl, acyloxy, alkoxy, or benzyl group or a glycosidic residue, and R³ means hydrogen or a phenylene residue of the general formula 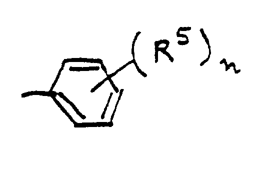 wherein R⁵ is halogen or a hydroxy, sulfonyl, alkyl, hydroxyalkyl, acyloxy or alkoxy group and n can have a value ranging from 0 to 3.

5. The use of any one of claims 1 or 4, wherein the benzopyrone derivatives are 1,4-benzopyrone derivatives of the formula 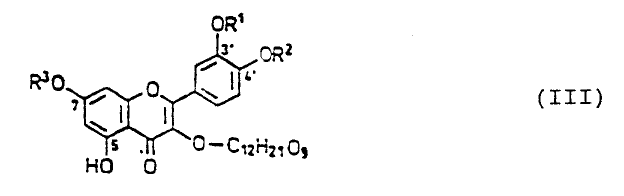 wherein R¹, R², R³ mean hydrogen or R¹ and R² are hydrogen and R³ is a (CH₂)₂-OH residue or R¹, R² and R³ are a (CH₂)₂-OH residue.

6. The use of any one of claims 1 or 4, wherein the benzopyrone is 1,4-benzopyrone of the formula

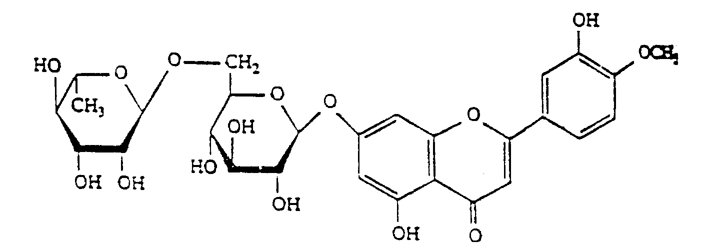

## Revendications

1. Utilisation de benzopyrones (benzo-α-pyrone et ses dérivés, benzo-γ-pyrone et ses dérivés) ou du dobésilate calcium (2,5-dihydroxybenzosulfonate) pour préparer un médicament destiné au traitement et à la prévention de maladies osseuses et articulaires dont la physio-pathologie correspond au principe du syndrome compartimental.

2. Utilisation selon la revendication 1, caractérisée en ce que les benzopyrones sont des dérivés de la 1,2-benzopyrone de la formule générale : 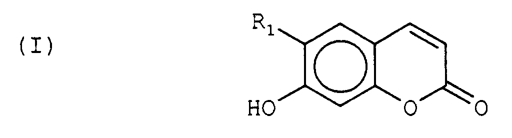 dans laquelle R₁ est un hydrogène, un halogène, un groupe hydroxyle, sulfonyle, alkyle, hydroxyalkyle, acyloxy, alcoxy ou benzyle, ou un résidu glycoside.

3. Utilisation selon la revendication 1 ou la revendication 2, caractérisée en ce que les benzopyrones sont des 1,2-benzopyrones des formules :

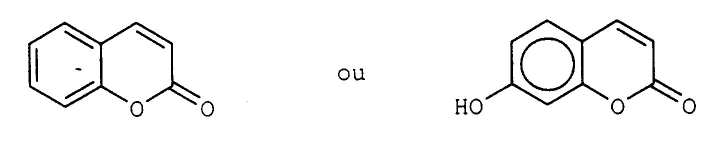

4. Utilisation selon la revendication 1, caractérisé en ce que les benzopyrones sont des 1,4-benzopyrones de la formule générale : 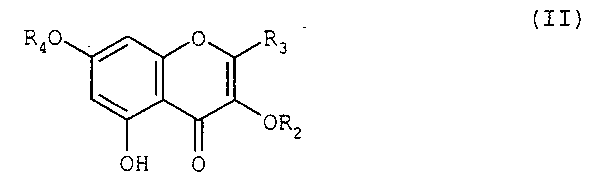 dans laquelle R₂ et R₄ sont indépendamment l'un de l'autre un hydrogène, un halogène, un groupe hydroxyle, sulfonyle, alkyle, hydroxyalkyle, acyloxy, alcoxy ou benzyle, ou un résidu glycoside, et R₃ représente un hydrogène ou un résidu phényle de la formule générale : 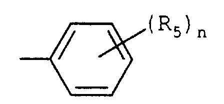 dans laquelle R₅ est un atome d'halogène, un groupe hydroxyle, sulfonyle, alkyle, hydroxyalkyle, acyloxy ou alcoxy, et n peut prendre des valeurs de 0 à 3.

5. Utilisation selon la revendication 1 ou la revendication 4, caractérisée en ce que les dérivés de benzopyrone sont des dérivés de la 1,4-benzopyrone de la formule : 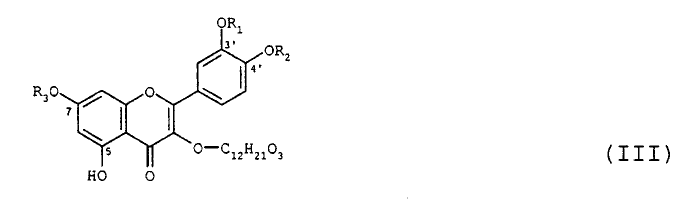 dans laquelle R₁, R₂ et R₃ sont chacun un hydrogène, ou bien R₁ et R₂ sont chacun un hydrogène et R₃ est un résidu (CH₂)₂-OH, ou bien R₁, R₂ et R₃ sont chacun un résidu (CH₂)₂-OH.

6. Utilisation selon la revendication 1 ou la revendication 4, caractérisé en ce que la benzopyrone est une 1,4-benzopyrone de la formule :
